Europäisches Patentamt

European Patent Office

Office européen des brevets

(19)

(11) Veröffentlichungsnummer : **0 167 581**
**B1**

(12) **EUROPÄISCHE PATENTSCHRIFT**

(45) Veröffentlichungstag der Patentschrift :
30.09.87

(21) Anmeldenummer : 85900470.7

(22) Anmeldetag : 21.12.84

(86) Internationale Anmeldenummer :
PCT/FI 84/00100

(87) Internationale Veröffentlichungsnummer :
WO/8502778 (04.07.85 Gazette 85/15)

(51) Int. Cl.⁴ : **A 61 M 15/00**

(54) INHALATIONSGERÄT.

(30) Priorität : 28.12.83 FI 834616

(43) Veröffentlichungstag der Anmeldung :
15.01.86 Patentblatt 86/03

(45) Bekanntmachung des Hinweises auf die Patenterteilung : 30.09.87 Patentblatt 87/40

(84) Benannte Vertragsstaaten :
AT BE CH DE FR GB LI NL SE

(56) Entgegenhaltungen :
DE-B- 1 046 264
NO-B-   134 730
SE-B-71 038 392
SE-B-79 014 171
US-A- 3 838 686

(73) Patentinhaber : HUHTAMÄKI OY
Lääketehdas Leiras P.O. Box 415
SF-20101 Turku (FI)

(72) Erfinder : LANKINEN, Tapio
Jalustinkatu 11
SF-20800 Turku (FI)

(74) Vertreter : Jackisch, Walter, Dipl.-Ing. et al
Menzelstrasse 40
D-7000 Stuttgart 1 (DE)

## Beschreibung

Die Erfindung betrifft ein Inhalationsgerät nach dem Oberbegriff des Patentanspruches 1. Ein derartiges Gerät ist z. B. aus der EP-A-0 015 247 bekannt.

Üblicherweise werden Medikamente unter Verwendung eines Inhalationsärosols oder einer Pulver-Dosiereinrichtung in die Lungen inhaliert. Die Benutzung von Inhalationsärosolen hat den schwerwiegenden Nachteil, daß das Medikament schon zu Beginn des Inhalierens richtig dosiert werden muß, wozu nicht alle Patienten in der Lage sind. Ein weiterer Nachteil besteht darin, daß Ärosole bei Temperaturen unter dem Nullpunkt infolge des relativ geringen Druckes der allgemein verwendeten Treibmittel nicht genügend verteilt werden.

Pulver-Dosiergeräte haben diese Nachteile nicht, erfordern jedoch infolge der kleinen Medikamentmengen einen Träger, wofür zur Zeit Laktose oder Glykose benutzt wird. Bei diesen Substanzen handelt es sich um hygroskopische Kohlehydrate, die zum Haften an feuchten Oberflächen neigen. Ein Pulver-Dosiergerät wird innen feucht, wenn es der Atemluft ausgesetzt oder aus der Kälte in warme Innenräume gebracht wird. Das führt zur Verschmutzung des Gerätes und Verringerung der inhalierten Dosis. Ungenaue Dosierung ist auch dann möglich, wenn das inhalationsbereite Gerät falsch gehalten wird, so daß ein Teil des Pulvers durch die Inhalationsöffnung herausfällt. Für den Benutzer ist das bei jeder Dosierung notwendige Befüllen eines Pulver-Dosiergerätes mit dem Medikament unbequem und schwierig.

Laktose und Glykose begünstigen Krankheitserreger im Mund, und infolge der fortgesetzten Einwirkung von im Pulver enthaltenen Zucker werden auch die Zähne angegriffen. Auch sind derartige Medikamente für gegen Laktose überempfindliche Patienten nicht immer geeignet.

Der Erfindung liegt die Aufgabe zugrunde, das Inhalationsgerät der eingangs genannten Art so auszubilden, daß es einfach zu bedienen ist und die wirksame Inhalation einer Substanz mit einem möglichst einfachen Verfahren ermöglicht.

Die Aufgabe wird gemäß der Erfindung nach den kennzeichnenden Merkmalen des Patentanspruches 1 gelöst.

Das erfindungsgemäße Gerät kombiniert die vorteilhaften Eigenschaften eines Ärosolsprühgerätes und eines Pulver-Inhalationsgerätes unter Vermeidung von deren Nachteilen. In dem Gerät wird das Medikament einem Ärosolbehälter entnommen, was für den Benutzer höchst wünschenswert und einfach ist, aber dennoch wird das Medikament wie bei der Inhalation eines Pulvers nach den individuellen Bedürfnissen des Patienten entsprechend dem Verhalten beim Inhalieren dosiert. Diese Art der Inhalation hat sich als wirksamer erwiesen als die Zuführung eines Ärosolsprays direkt in den Mund.

Die erforderliche Inhalationsluft strömt der Inhalationskammer über das Rückschlagventil zu, das entweder nur durch das Ansaugen des versprühten Medikamentes über das Mundstück oder auch zusätzlich von Hand zu öffnen ist. Zweckmäßig befindet sich das Rückschlagventil in einer Zwischenwand zwischen Stutzen und Inhalationskammer, so daß es geschützt unter dem Ärosolbehälter liegt. Der Stutzen kann mit einer Schutzabdeckung versehen sein.

Um die bestmögliche Zerstäubung zu erzielen, ist die der Düse zugewandte Fläche der Prallplatte konkav. Dadurch wird der Sprühstrahl wirksam zerstäubt und breitet sich in der ganzen Kammer aus.

Weitere Merkmale der Erfindung ergeben sich aus den Unteransprüchen.

Mehrere Ausführungsformen des erfindungsgemäßen Inhalationsgerätes werden im folgenden anhand der Zeichnungen beschrieben. Es zeigen :

Figur 1    das Inhalationsgerät schematisch im Axialschnitt,

Figuren 2 bis 4    verschiedene Abwandlungen dieses Gerätes.

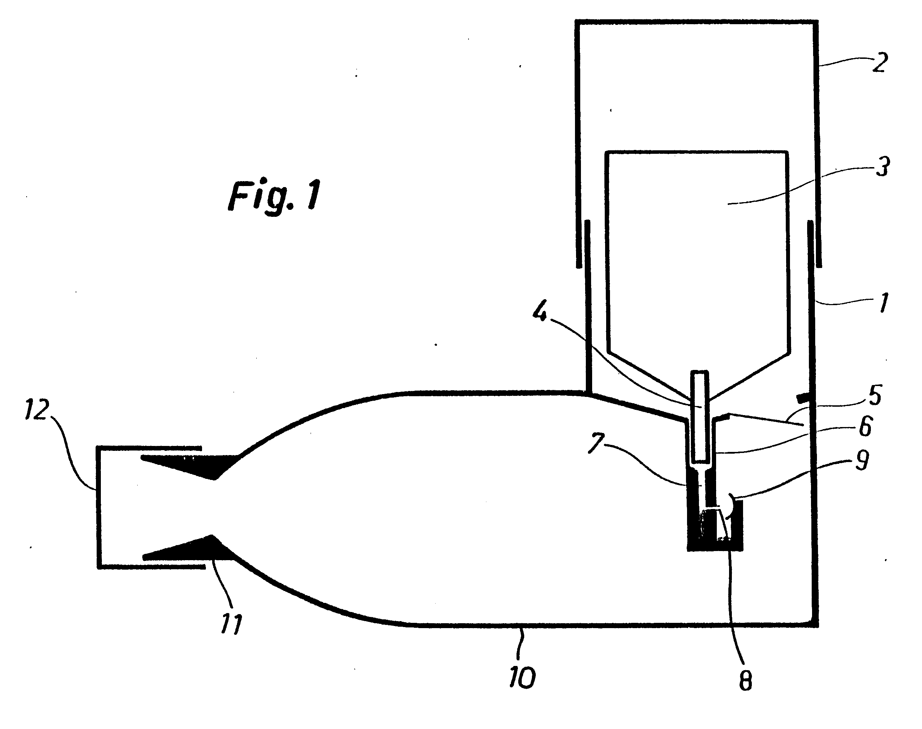

Das in Fig. 1 gezeigte Gerät hat ein rohrförmiges Gehäuse, das eine Inhalationskammer 10 bildet und an seinem einen Ende einen nach oben gerichteten Stutzen 1 aufweist. Dieser Stutzen 1 ist zur Aufnahme eines Ärosolbehälters 3 für ein Medikament vorgesehen. Der Boden des Stutzens 1 ist einstückig mit einem Verbindungsstück 6 ausgebildet, das einen Kanal enthält, dessen Einlaß genügend weit ist, um einen Ventilschaft 4 des eingesetzten Ärosolbehälters 3 aufnehmen zu können. Unterhalb der Einlaßöffnung ist der Kanal verengt und bildet dadurch einen Begrenzungsanschlag für den Ventilschaft 4. Die Verengung bildet einen Kanal 7, der koaxial in dem Verbindungsstück 6 verläuft. Am Ende dieses Kanals 7 ist eine zu ihm senkrechte Querbohrung vorgesehen, die in eine Düse 8 mündet.

Der Düse 8 liegt mit Abstand eine Prallplatte 9 gegenüber, deren konkave Oberfläche der Düse 8 zugewandt ist.

Der Stutzen 1 ist zur Aufnahme des Ärosolbehälters 3 nach oben offen. Eine aufgesetzte Schutzkappe 2 deckt den Ärosolbehälter 3 ab.

Zwischen der vom Gehäuse gebildeten Inhalationskammer 10 und dem Stutzen 1 liegt eine Wandung, die in ihrer Mitte das Verbindungsstück 6 und seitlich daneben ein Rückschlagventil 5 enthält, das nur zur Inhalationskammer 10 hin öffnet und das Einströmen von Luft in die Inhalationskammer ermöglicht. Bei entgegengesetztem Luftstrom schließt das Ventil 5.

Die Inhalationskammer ist rohrförmig, beispielsweise zylindrisch, und an ihrem vom Stutzen 1 abgewandten Ende mit einem Mundstück 11 versehen, das im oder vor dem Mund des Benutzers zu halten ist und mit einer Schutzkappe 12 verschlossen werden kann.

Zur Benutzung des Gerätes wird der mit einem Medikament gefüllte Ärosolbehälter 3 in den Stutzen 1 eingesetzt, bis der Ventilschaft 4 auf dem von der stirnseitigen Begrenzung des Kanals 7 gebildeten Boden des oberen, im Querschnitt größeren Kanalstückes aufsitzt. Durch Druck auf den Behälter 3 von oben wird der Ventilschaft 4 in den Behälter verschoben und das zugehörige Dosierventil geöffnet, so daß eine bestimmte Menge an Gas und Medikamentsuspension in den Kanal 7 entweicht, durch die Düse 8 austritt und die Prallplatte 9 anströmt. Dadurch wird das zu vergasende Gemisch in die Inhalationskammer abgelenkt, und die Vergasung setzt im hinteren Teil der Inhalationskammer 10 ein. Das expandierende Gas verdrängt die vor ihm liegende Luft zu dem Mundstück 11. Während der Expansion verhindert das Rückschlagventil 5, daß Gas auf diesem Wege die Kammer 10 verläßt. Die freigesetzte Gasmenge ist so bemessen, daß sie bei normalem Druck nur einen Teil der Inhalationskammer ausfüllt. Partikel des Medikamentes bilden zusammen mit dem Gas ein Gemisch. Wenn der Benutzer das offene Mundstück 11 in den Mund nimmt und inhaliert, öffnet das Rückschlagventil 5 und läßt Luft in die Inhalationskammer 10 einströmen. Die strömende Luft nimmt das zerstäubte Medikament in der Kammer mit und befördert es in die Lungen des Benutzers.

Bedingt durch das Arbeitsprinzip sind bei dem erfindungsgemäßen Gerät der Treibmitteldruck und die Größe der Düsenöffnung weniger kritsch als bei einem üblichen Ärosolgerät. Durch Erhöhung des Gasdruckes kann das Gerät auch bei Temperaturen unter dem Nullpunkt benutzt werden. Der Durchmesser der Düsenöffnung bestimmt die Vergasungszeit derart, daß so viel Medikament wie möglich inhaliert werden kann.

Die Behälter-Schutzkappe 2 dient zum Schutz des Gerätes und insbesondere des Ärosolbehälters 3 bei der Beförderung. Da auch das Mundstück 11 durch die Schutzkappe 12 abgeschlossen wird, ist das Gerät beim Transport vollkommen verschlossen und gegen Verunreinigungen abgeschirmt.

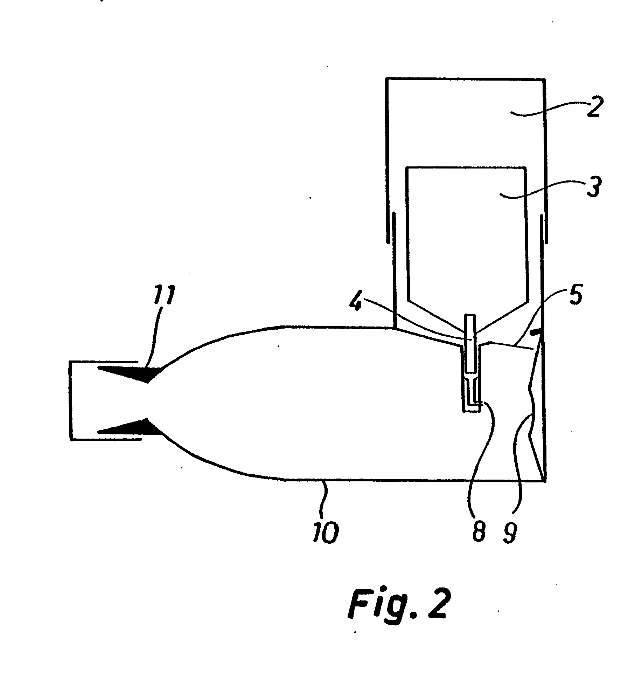

Die Fig. 2 bis 4 zeigen verschiedene Abwandlungen des Gerätes nach Fig. 1. Die mit dem Gerät nach Fig. 1 übereinstimmenden Teile sind gleich bezeichnet und nochmals erläutert.

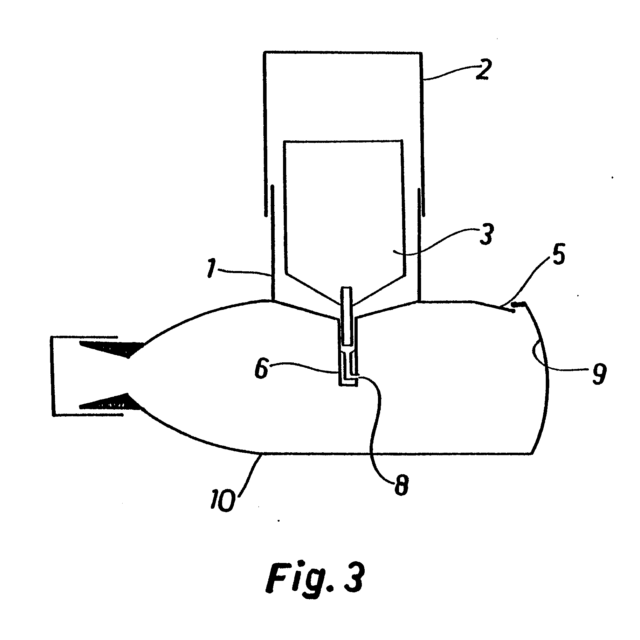

Bei der Ausführungsform nach Fig. 2 ist an der dem Mundstück 11 gegenüberliegenden Gehäusewand ein Ansatzstück befestigt, das eine zentrische Vertiefung zur Bildung einer konkaven Prallplatte 9a aufweist, die der Düse 8 zugewandt ist. Fig. 3 zeigt eine Ausführung, bei der die dem Mundstück gegenüberliegende Stirnwand des Gehäuses selbst als konkave Prallplatte 9b ausgeführt ist. In diesem Fall sind der Stutzen 1 und sein Verbindungsstück 6 in der Längsmitte der Inhalationskammer 10 angeordnet, wobei die Düse 8 einen relativ großen Abstand von der Prallplatte 9 hat. Ein Rückschlagventil 5a ist in der Nähe der Prallplatte 9b in der Gehäusewandung vorgesehen, so daß es die Kammer 10 beim Inhalieren direkt mit der Umgebungsluft verbindet und der Stutzen 1 mittels der Kappe 2 dicht verschlossen bleiben kann.

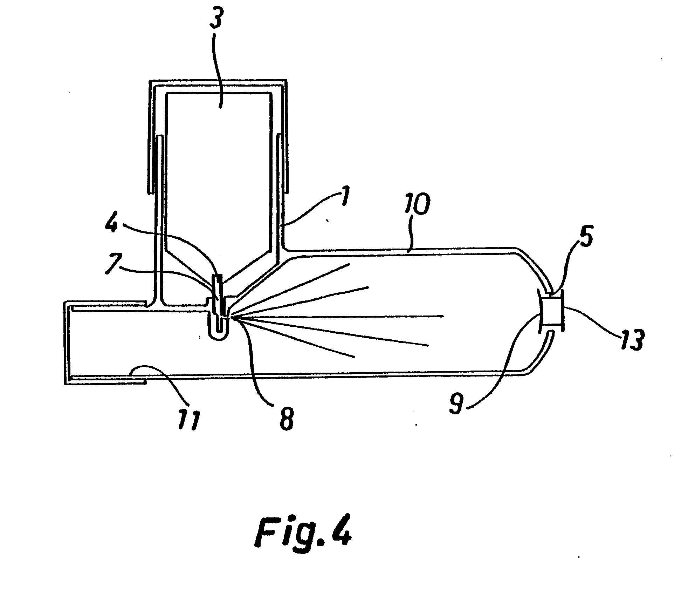

Das Gerät nach Fig. 4 hat ein rohrförmiges Gehäuse, das im vorderen Abschnitt bis zur Mittelachse des Stutzens 1 im Durchmesser reduziert ist und dadurch das Mundstück 11a bildet, das an die im Querschnitt größere Inhalationskammer 10 anschließt, wobei der Stutzen, wie bei der Ausführungsform nach Fig. 3, mit der Kammer nur über den Kanal 7 und die Düse 8 in Verbindung steht. Der Stutzen 1 befindet sich im vorderen Teil des Gerätes, so daß die Düse 8 im vorderen Bereich der Kammer 10 liegt. Die Rückwand des Gehäuses bzw. der Kammer 10 ist gewölbt, wobei ihre konkave Seite der Düse 8 zugewandt ist. In der Rückwand ist eine zentrale Öffnung vorgesehen, die zusammen mit einem in sie eingesetzten Ventilstück 13 ein Rückschlagventil 5b bildet. Das Ventilstück 13 trägt innerhalb der Kammer 10 eine konkave Prallplatte 9c, welche die Öffnungen in der Kammerrückwand dicht verschließt, wenn der Sprühstrahl aus der Düse 8 auf sie auftrifft.

Zum Inhalieren kann das Ventilstück 13 in der Öffnung von Hand nach innen gedrückt werden, wobei der Verschiebeweg durch äußere Anschlagnasen oder einen Ringflansch des Ventilstückes begrenzt ist. Durch das Einschieben des Ventilstückes wird das Ventil 5b geöffnet, so daß in die Inhalationskammer 10 Luft einströmen kann.

## Patentansprüche

1. Inhalationsgerät mit einem zur Aufnahme eines Ärosolbehälters (3) bestimmten Stutzen (1), der ein in eine Inhalationskammer (10) mündendes Verbindungsstück (6) aufweist, in das der Ventilschaft (4) eines Dosierventils des Ärosolbehälters (3) einsetzbar ist und das einen Einlaßkanal (7) mit einer Düse (8) aufweist, die in die Inhalationskammer (10) mündet, welche mit einem Mundstück (11) versehen ist, dadurch gekennzeichnet, daß die Düse (8) des Einlaßkanals (7) auf das vom Mundstück (11) abgewandte Ende der Inhalationskammer (10) gerichtet ist, daß der Düse (8) mit Abstand eine Prallplatte (9) zum Zerstäuben des Medikamenten-Sprühstrahls in der Inhalationskammer (10) gegenüberliegt, und daß die Inhalationskammer (10) ein Rückschlagventil (5) zum Einlassen von Luft aufweist.

2. Gerät nach Anspruch 1, dadurch gekennzeichnet, daß das Rückschlagventil (5) in einer zwischen dem Stutzen (1) und der Inhalationskammer (10) befindlichen Zwischenwand angebracht ist.

3. Gerät nach Anspruch 1, dadurch gekennzeichnet, daß das Rückschlagventil (5a ; 5b) in einer Außenwand der Inhalationskammer (10) angeordnet ist.

4. Gerät nach Anspruch 3, dadurch gekennzeichnet, daß das Rückschlagventil (5b) mittels eines von Hand in die Inhalationskammer (10) zu bewegenden Ventilstückes (13) zu öffnen ist.

5. Gerät nach einem der Ansprüche 1 bis 4, dadurch gekennzeichnet, daß die der Düse (8) zugewandte Fläche der Prallplatte (9) konkav ist.

6. Gerät nach einem der Ansprüche 1 bis 5, dadurch gekennzeichnet, daß sich die Prallplatte (9a ; 9b ; 9c) an der dem Mundstück (11 ; 11a) gegenüberliegenden Wand der Inhalationskammer (10) befindet.

7. Gerät nach einem der Ansprüche 4 bis 6, dadurch gekennzeichnet, daß die Prallplatte (9c) Bestandteil des von Hand in die Inhalationskammer (10) verschiebbaren Ventilstückes (13) ist.

8. Gerät nach einem der Ansprüche 1 bis 7, dadurch gekennzeichnet, daß der Stutzen (1) und das Mundstück (11) jeweils durch eine Abdeckkappe dicht verschließbar sind.

## Claims

1. Inhalation device with a socket (I) intended for receiving an aerosol container (3), which socket comprises a connecting member (6) opening into an inhalation chamber (10), into which connecting members (6) the valve stem (4) of a metering valve of the aerosol container (3) can be inserted and which comprises an inlet duct (7) with a nozzle (8), which opens into the inhalation chamber (10), which is provided with a mouthpiece (11), characterised in that the nozzle (8) of the inlet duct (7) directed towards the end of the inhalation chamber (10) remote from the mouthpiece (11), that located opposite the nozzle (8) at a distance therefrom is a baffle plate (9) for atomizing the spray jet of medication in the inhalation chamber (10) and that the inhalation chamber (10) comprises a one-way valve (5) for the admission of air.

2. Device according to Claim 1, characterised in that the one-way valve (5) is disposed in an intermediate wall located between the socket (1) and the inhalation chamber (10).

3. Device according to Claim 1, characterised in that the one-way valve (5a ; 5b) is disposed in an outer wall of the inhalation chamber (10).

4. Device according to Claim 3, characterised in that the one-way valve (5b) can be opened by means of a valve member (13) to be moved by hand in the inhalation chamber (10).

5. Device according to one of Claims 1 to 4, characterised in that the surface of the baffle plate (9) facing the nozzle (8) is concave.

6. Device according to one of Claims 1 to 5, characterised in that the baffle plate (9a ; 9b ; 9c) is located on the wall of the inhalation chamber (10) lying opposite the mouth-piece (11 ; 11a).

7. Device according to one of Claims 4 to 6, characterised in that the baffle plate (9c) is part of the valve member (13) able to be moved by hand in the inhalation chamber (10).

8. Device according to one of Claims 1 to 7, characterised in that the socket (1) and the mouth-piece (11) can each be closed off in an airtight manner by a cover.

## Revendications

1. Dispositif ou appareil inhalateur équipé d'une tubulure (1) qui, destiné à recevoir un réservoir à aérosol (3), comporte un élément de mise en communication (6) débouchant dans une chambre d'inhalation (10) et dans lequel peut être engagée la tige de soupape (4) d'une valve doseuse du réservoir à aérosol (3), lequel élément de mise en communication présente un canal d'admission (7) avec un ajutage (8) qui débouche dans la chambre d'inhalation (10), laquelle est munie d'une embouchure (11), caractérisé en ce que l'ajutage (8) du canal d'admission (7) est orienté vers l'extrémité de la chambre d'inhalation (10) éloignée de l'embouchure (11), en ce qu'en regard de l'ajutage (8) se trouve à une certaine distance de celui-ci une plaque de rebondissement (9) destinée à pulvériser dans la chambre d'inhalation (10) le jet de médicament projeté et en ce que la chambre d'inhalation (10) présente un clapet de retenue (5) pour l'admission d'air.

2. Appareil selon la revendication 1, caractérisé en ce que le clapet de retenue (5) est disposé dans une paroi intermédiaire située entre la tubulure (1) et la chambre d'inhalation (10).

3. Appareil selon la revendication 1, caractérisé en ce que le clapet de retenue (5a ; 5b) est disposé dans une paroi extérieure de la chambre d'inhalation (10).

4. Appareil selon la revendication 3, caractérisé en ce que le clapet de retenue (5b) doit être ouvert au moyen d'un obturateur (13) à pousser manuellement dans la chambre d'inhalation (10).

5. Appareil selon l'une quelconque des revendications précédentes, caractérisé en ce que la face de la plaque de rebondissement (9) située en regard de l'ajutage (8) est concave.

6. Appareil selon l'une quelconque des revendications précédentes, caractérisé en ce que la plaque de rebondissement (9a ; 9b ; 9c) se trouve au niveau de la paroi de la chambre d'inhalation (10) opposée à l'embouchure (11 ; 11a).

7. Appareil selon l'une quelconque des revendications 4 à 6, caractérisé en ce que la plaque de rebondissement (9c) fait partie intégrante de l'obturateur (13) pouvant être poussé avec la main dans la chambre d'inhalation (10).

8. Appareil selon l'une quelconque des revendications précédentes, caractérisé en ce que la tubulure (1) et l'embouchure (11) peuvent chacune être fermées de manière étanche par une coiffe protectrice.

*Fig.1*

**Fig. 2**

0 167 581

**Fig. 3**

*Fig.4*

0 167 581